# FASCICULE DE BREVET EUROPEEN

(11) **EP 4 319 857 B1**
(45) Date de publication et mention de la délivrance du brevet: **18.11.2026**
(21) Numéro de dépôt: 22719979.1
(22) Date de dépôt: 06.04.2022
(51) Int. Cl.: A61M 29/02, A61M 25/10, A63B 23/20, A61B 5/107, A61B 5/00, A63B 21/008

(54) **SYSTÈME POUR LA PRÉPARATION DES VOIES GÉNITALES D'UNE FEMME AVANT UN ACCOUCHEMENT**
SYSTEM ZUR VORBEREITUNG DES GENITALTRAKTES EINER FRAU VOR DER GEBURT
SYSTEM FOR PREPARING THE GENITAL TRACT OF A WOMAN PRIOR TO CHILDBIRTH

(30) Priorité: 07.04.2021 FR 2103560
(43) Date de publication de la demande: 14.02.2024
(73) Titulaire: Mumming, 33323 Bègles Cedex (FR)
(72) Inventeur: ZUBER, Thomas, 33000 Bordeaux (FR); MELLERIO, Gonzague, 75009 Paris (FR)
(74) Mandataire: Germain Maureau
(86) Numéro de dépôt international: PCT/FR2022/050642
(87) Numéro de publication internationale: WO 2022/214767

(56) Documents cités:
- WO-A1-2021/058462
- CN-A- 107 049 342
- FR-A1- 2 365 327
- US-A1- 2003 087 734

## Description

### Domaine Technique

La présente invention se rapporte à un système destiné à préparer les voies génitales d'une femme avant un accouchement. En particulier, l'invention se rapporte à un système connecté comprenant un corps gonflable destiné à être placé dans les voies génitales de la femme en vue d'assouplir les voies génitales, un dispositif de mesure configuré pour mesurer une dimension du corps gonflable et dont les données de mesures de dimension sont collectées en temps réel pour permettre à la femme de suivre son évolution.

### Technique antérieure

Lors d'un accouchement naturel, les voies génitales (comprenant le vagin, le plancher pelvien et le périnée) d'une femme sont soumises à un étirement pouvant causer des dommages indésirables.

Dans certains cas, une épisiotomie est réalisée pour faciliter le passage du bébé.

Cet acte chirurgical peut avoir des conséquences importantes pour la femme telles que des douleurs physiques et psychiques plus ou moins intenses et peut également nécessiter un certain temps de cicatrisation.

Pour pallier à ces inconvénients, il existe des exercices de préparation à l'accouchement qui vont permettre de préparer les voies génitales au passage du bébé au cours de l'accouchement.

Il existe différents types de système permettant de réaliser ces exercices. On connait par exemple le système connu sous le nom d'Epi-no^{®} qui comprend un corps gonflable destiné à être placé dans les voies génitales d'une utilisatrice et qui est configuré pour être gonflé manuellement au moyen d'une poire de gonflage reliée au corps gonflable par un conduit. En fonctionnement, l'utilisatrice gonfle le corps gonflable au moyen de la poire de gonflage. Le corps gonflable exerce une force sur les muscles des voies génitales de l'utilisatrice et permet un assouplissement progressif des voies génitales de l'utilisatrice. L'expansion du corps gonflable peut s'intensifier progressivement au cours des séances de préparation.

Un inconvénient de ce système est qu'il n'est pas possible de quantifier de manière fiable l'expansion de corps gonflable et donc de pouvoir suivre avec précision l'évolution de l'assouplissement des voies génitales.

Un autre inconvénient de ce système est qu'il n'est pas possible pour l'utilisatrice d'obtenir des informations relatives à l'évolution de sa préparation en temps réel et de manière simple.

US 2003/087734 décrit un système d'exercice prénatal de dilatation du vagin. Le ballon formant un creux pour ne pas appuyer sur l'urètre .

### Exposé de l'invention

Le présent exposé a pour but de résoudre tout ou partie des inconvénients mentionnés ci-dessus. L'invention est définie dans la revendication indépendante 1. D'autres aspects de l'invention sont définis dans les revendication dépendantes 2-14.

Le présent exposé concerne un système pour la préparation des voies génitales d'une femme avant selon la revendication 1.

Le système selon l'invention permet la préparation musculaire, cutanée et des muqueuses à l'accouchement pour éviter les lésions et leurs conséquences.

Le système selon le présent exposé permet de préparer les voies génitales de la femme afin de faciliter l'accouchement.

L'invention permet en outre un suivi en temps réel de l'évolution par l'utilisatrice et un meilleur contrôle de son périnée.

Selon un aspect la paroi du corps gonflable présente une portion tubulaire de section constante ou variable.

Selon un mode de réalisation, le dispositif de contrôle est agencé pour transmettre les données de mesure de dimension du dispositif de mesure à un dispositif d'interface configuré pour recevoir et afficher des données de mesure de dimension du dispositif de mesure, notamment par une liaison de communication sans fil.

Selon un mode de réalisation, le dispositif d'interface comprend une application informatique exécutée sur un terminal de communication mobile. Le système peut comprendre le dispositif d'interface. Alternativement, le dispositif d'interface peut être constitué par une application ou un dispositif tiers agencé pour traiter les données de mesure de dimension du dispositif de mesure.

L'invention permet de fournir un système dont les dimensions du corps gonflable sont connues en temps réel et retransmises à l'utilisatrice par l'intermédiaire par exemple d'un objet disposant d'un écran (téléphone, tablette, ordinateur...).

Un avantage de l'invention réside dans la capacité à lire et retranscrire en temps réel les données d'utilisation du corps gonflable afin d'orienter l'utilisatrice vers une bonne utilisation du dispositif et ainsi améliorer sa progression.

Selon un mode de réalisation, le dispositif de mesure est configuré pour mesurer une dimension du corps gonflable correspondant à une section transversale du corps gonflable.

On entend par section transversale une section du corps gonflable considérée de façon transversale à une direction d'insertion du corps gonflable dans les voies génitales.

Selon l'invention, le corps gonflable présente une section transversale sensiblement circulaire lorsque le corps est gonflé. Dans ce mode de réalisation, le système est agencé pour déterminer un diamètre de ladite section transversale du corps gonflable.

Selon un mode de réalisation, le dispositif de mesure est intégré dans le corps gonflable et comprend au moins un capteur de distance. Dans ce mode de réalisation, le corps gonflable comprend un support central s'étendant dans le corps gonflable et le capteur de distance est agencé sur le support central du corps gonflable, le dispositif de mesure étant configuré pour mesurer une distance entre le support central et un point localisé sur une face interne de la paroi du corps gonflable.

Selon une caractéristique, le support central est une tige semi-rigide et ledit au moins un capteur est agencé sur une extrémité libre de la tige semi-rigide.

Selon une caractéristique, ledit au moins un capteur de distance est un capteur choisi parmi le groupe constitué par un capteur infrarouge, un capteur ultrason et un capteur laser.

Selon une caractéristique, ledit au moins un capteur de distance est un appareil de prise de vue et le corps gonflable comprend au moins un motif ou une nervure agencé sur une face interne de la paroi du corps gonflable.

Par exemple l'appareil de prise de vue est une caméra, un appareil photo digital.

Selon un mode de réalisation, le système comprend une première partie destinée à être placée dans les voies génitales et une deuxième partie destinée à rester en extérieur des voies génitales. La première partie comprend le corps gonflable et la deuxième partie comprend le dispositif de gonflage, le dispositif de contrôle et le dispositif de mesure, le dispositif de mesure comprenant au moins un capteur de pression et au moins un calculateur configuré pour calculer une quantité d'air entrant dans le corps gonflable et/ou sortant du corps gonflable.

Selon une caractéristique, le dispositif comprend au moins un capteur de température.

Ainsi, le dispositif de mesure est agencé pour déterminer la dimension du corps gonflable en fonction d'une valeur de pression, d'une valeur de température et d'une quantité d'air dans le corps gonflable. Il est à noter que le dispositif de mesure peut comprendre à cet effet une unité de traitement qui peut être distincte ou partagée avec le dispositif de contrôle et/ ou avec le dispositif de mesure.

Selon une caractéristique, le calculateur est configuré pour calculer une quantité d'air entrant dans le corps gonflable utilisant des informations communiquées par un moteur.

Selon une caractéristique, le calculateur est configuré pour calculer une quantité d'air sortant du corps gonflable utilisant des informations communiquées par une électrovanne.

Selon une caractéristique, la deuxième extrémité du corps gonflable comprend un renflement.

Selon une caractéristique, le système comprend un moyen de préhension destiné à permettre un positionnement du corps gonflable dans les voies génitales de la femme, le moyen de préhension étant un manche semi-rigide.

Selon une caractéristique, le manche semi-rigide est télescopique.

D'autres caractéristiques et avantages de l'invention apparaîtront à la lecture de la description nullement limitative qui suit et des figures annexées qui illustrent de manière schématique plusieurs modes de réalisation du système selon l'invention.

### Brève description des dessins

[Fig. 1] La figure 1 est une illustration d'un système de préparation à l'accouchement selon un mode de réalisation de l'invention.
[Fig. 2] La figure 2 est une vue agrandie d'un corps gonflable du système de préparation selon l'invention.
[Fig. 3] La figure 3 illustre le système comprenant un dispositif de mesure selon un premier mode de réalisation de l'invention.
[Fig. 4] La figure 4 illustre une variante du dispositif de mesure selon le premier mode de réalisation.
[Fig. 5] La figure 5 illustre le système comprenant un dispositif de mesure selon un deuxième mode de réalisation de l'invention.
[Fig. 6] La figure 6 illustre le corps gonflable du système selon une variante de réalisation.
[Fig. 7] La figure 7 illustre le corps gonflable du système selon une variante de réalisation.
[Fig. 8] La figure 8 illustre le corps gonflable du système selon une variante de réalisation.
[Fig. 9] La figure 9 illustre le corps gonflable du système selon une variante de réalisation.
[Fig. 10] La figure 10 illustre le corps gonflable du système selon une variante de réalisation.
[Fig. 11] La figure 11 illustre le corps gonflable du système selon une variante de réalisation.
[Fig. 12] La figure 12 illustre le corps gonflable du système selon une variante de réalisation.

### Description des modes de réalisation

Par souci de simplification, les éléments identiques sont repérés par des signes de référence identiques sur l'ensemble des figures.

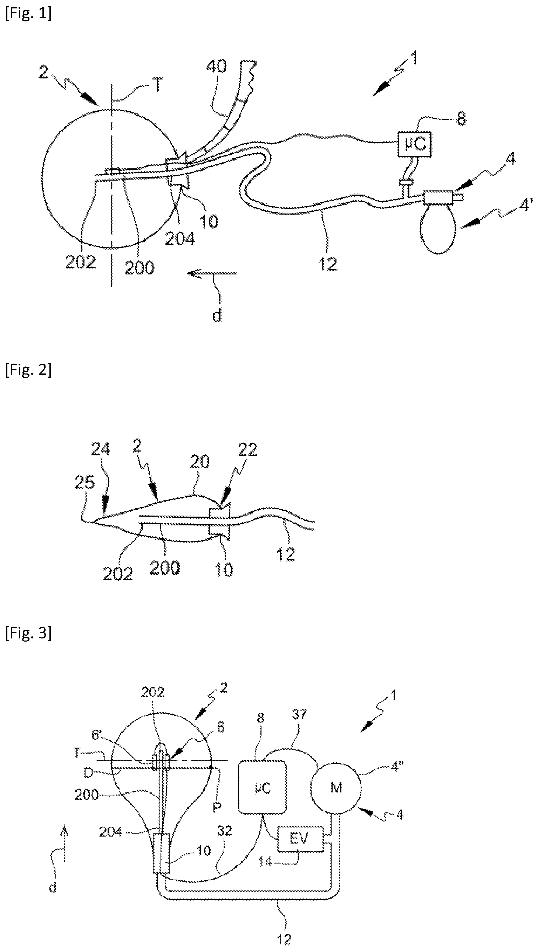

La figure 1 illustre un système 1 pour la préparation à l'accouchement d'une femme selon le présent exposé.

Le système 1 comprend un corps gonflable 2 destiné à être placé dans les voies génitales de la femme, un dispositif de gonflage 4 destiné au gonflage du corps gonflable, un dispositif de mesure 6 configuré pour mesurer une dimension du corps gonflable et un dispositif de contrôle 8 configuré pour collecter les données de mesure de dimension du dispositif de mesure 6.

Le corps gonflable 2 est configuré pour exercer une force sur les muscles des voies génitales de la femme dans le but d'assouplir les voies géniales lorsque le corps est gonflé, par exemple au moyen d'un gaz tel que l'air.

Le corps gonflable 2 comprend un matériau compatible avec le corps humain, par exemple le corps gonflable 2 peut comprendre du latex ou du silicone.

Comme l'illustre la figure 2, le corps gonflable 2 comprend une paroi 20 comportant une première extrémité 22 ouverte destinée à permettre une entrée d'air dans le corps gonflable ou une sortie d'air du corps gonflable et une deuxième extrémité 24 fermée opposée à la première extrémité 22.

Selon un aspect la paroi du corps gonflable présente une portion de section constante ou variable.

Par exemple la paroi du corps gonflable présente une portion tubulaire de section constante ou variable.

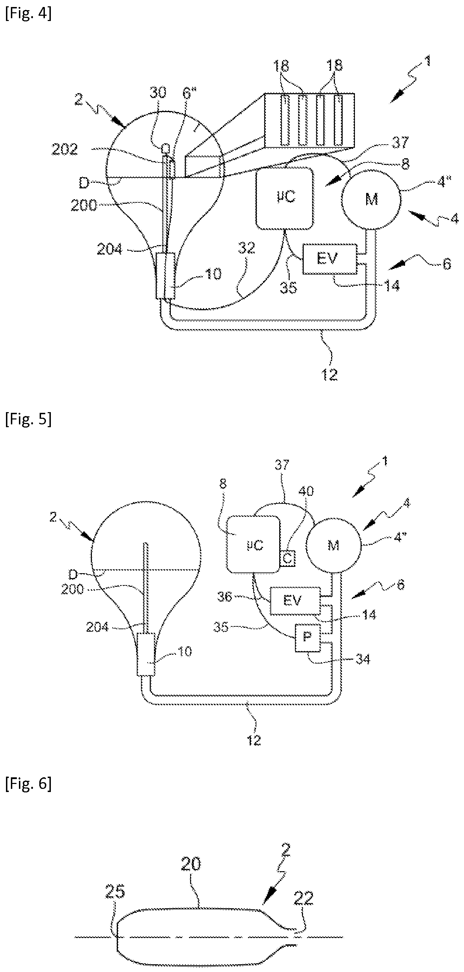

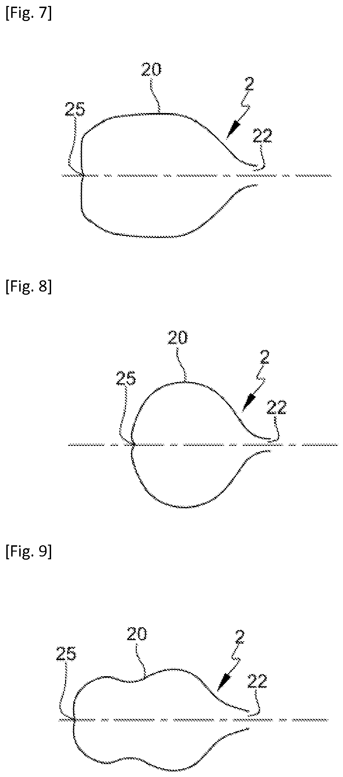

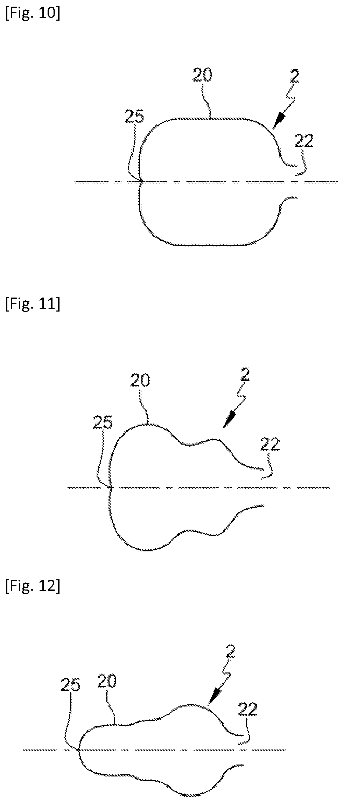

Des exemples de formes de la paroi sont présentées dans les figures 6 à 12 qui illustrent des vues en coupe longitudinales du corps gonflable selon plusieurs variantes de réalisation.

Dans l'exemple représenté, la deuxième extrémité 24 peut comprendre un renflement 25. Le renflement 25 permet une fixation d'un élément lubrifié sur le corps gonflable pour permettre de faciliter une insertion du corps gonflable dans les voies génitales de l'utilisatrice.

Le corps gonflable 2 peut comprendre un support central 200 s'étendant dans le corps gonflable 2. Par exemple, le support central 200 est une tige semi-rigide qui permet de faciliter l'insertion du corps gonflable 2 dans les voies génitales de la femme. La tige semi- rigide comprend une première extrémité 202 libre s'étendant au niveau d'une section médiane du corps gonflable 2 et une deuxième extrémité 204 reliée à l'extrémité ouverte 22 du corps gonflable 2. Par exemple la tige semi-rigide est fixée sur un col formant une poignée 10 sur laquelle est fixé le corps gonflable 2. La poignée 10 permet en outre de guider l'insertion du corps gonflable 2 dans les voies génitales.

Lorsque le corps est gonflé, le corps présente une section transversale « T » sensiblement circulaire comme l'illustre les figures 1, 3, 4 et 5.

On entend par section transversale « T » une section du corps gonflable considérée de façon transversale à une direction d'insertion « d » du corps gonflable dans les voies génitales. Dans cette configuration, le corps gonflable permet en outre de simuler un passage d'une tête d'un bébé.

Le corps gonflable comprend une épaisseur déterminée pour que le corps gonflable ne se dégonfle pas même si l'utilisatrice exerce une force sur le corps gonflable. Le système permet ainsi à l'utilisatrice d'exercer ces muscles en appliquant une pression sur le corps gonflable pendant un exercice par exemple.

Le corps gonflable 2 est gonflé au moyen du dispositif de gonflage 4 qui est relié fluidiquement à l'extrémité ouverte 204 du corps gonflable 2 par un conduit 12 tel qu'un tuyau souple.

Selon un premier mode de réalisation illustré à la figure 1, le dispositif de gonflage 4 comprend une poire manuelle de gonflage 4'. Dans ce mode de réalisation, la poire manuelle peut comprendre une soupape (non représentée) permettant de dégonfler légèrement ou en totalité le corps gonflable 2.

Selon un deuxième mode de réalisation illustré aux figures 3 à 5, le dispositif de gonflage 4 est un dispositif motorisé et comprend un moteur 4".

Le moteur fonctionne comme une pompe à air.

Par exemple le moteur est un moteur électrique solénoïde comportant un adaptateur destiné à pomper l'air de l'atmosphère via une ouverture prévue à cet effet.

Dans ce deuxième mode de réalisation, le système comprend une électrovanne 14 permettant de dégonfler légèrement ou en totalité le corps gonflable 2. Le moteur 4" et l'électrovanne 12 sont reliés au dispositif de contrôle par des fils conducteurs 36, 37.

Le dispositif de mesure 6 est configuré pour mesurer une dimension du corps gonflable au cours du gonflage du corps. En particulier, le dispositif de mesure est configuré pour mesurer une dimension du corps correspondant à la section transversale « T » du corps gonflable 2.

En outre, le dispositif de mesure 6 permet au système 1 d'être agencé pour déterminer un diamètre « D » de la section transversale circulaire du corps gonflable en fonction des mesures mesurées par le dispositif de mesure 6.

Les données de mesure de dimension du dispositif de mesure 6 sont en outre collectées par le dispositif de contrôle 8.

Le dispositif de contrôle 8 comprend par exemple un microcontrôleur. Le système comprend une batterie (non représentée) destinée à alimenter le microcontrôleur.

Le dispositif de contrôle 8 est agencé pour transmette les données de mesure de dimension du dispositif de mesure à un dispositif d'interface (non représenté) configuré pour recevoir et afficher des données de mesure de dimension du dispositif de mesure, notamment par une liaison de communication sans fil.

Selon un mode de réalisation, le dispositif d'interface comprend une application informatique exécutée sur un terminal de communication mobile.

Le système peut comprendre le dispositif d'interface.

Alternativement, le dispositif d'interface peut être constitué par une application ou un dispositif tiers agencé pour traiter les données de mesure de dimension du dispositif de mesure.

Le système permet d'afficher les informations à l'utilisatrice en temps réel et peut également les comparer instantanément à des mesures habituellement observées.

Afin de proposer des informations plus pertinentes encore, le système peut être agencé pour permettre à l'utilisatrice de rentrer des informations sur sa grossesse (nombre de semaines d'aménorrhée, âge, poids, taille, pointure). Si l'utilisatrice a intégré ces données au système, le système comparera alors les mesures collectées en temps réel à celles habituellement observées pour une personne du même âge, du même gabarit et au même moment de sa grossesse.

Selon un premier mode de réalisation illustré aux figures 3 et 4, le dispositif de mesure 6 est intégré dans le corps gonflable 2.

Dans ce mode de réalisation, le dispositif de mesure comprend au moins un capteur de distance 6'.

Dans les exemples représentés, un capteur est agencé sur le support central 200 du corps gonflable 2.

Par exemple, lorsque le support central 200 est une tige semi-rigide, le capteur de distance 6' est agencé sur l'extrémité libre 202 de la tige du corps gonflable.

Le dispositif de mesure 6 est configuré pour mesurer une distance entre le support central 200 et un point « P » localisé sur une face interne de la paroi 20 du corps gonflable.

En outre cette distance correspond à un rayon du corps gonflable.

Par exemple, le capteur de distance 6' est un capteur infrarouge.

Le capteur infrarouge comprend un émetteur et un récepteur. L'émetteur est configuré pour émettre une lumière infrarouge en direction de la paroi du corps gonflable qui est retourné par le corps gonflable et le récepteur est configuré pour mesurer une quantité d'énergie retournée par le corps gonflable.

Selon un autre exemple, le capteur de distance 6' est un capteur ultrason.

Le capteur ultrason comprend un émetteur et un récepteur. L'émetteur est configuré pour émettre un signal ultrason en direction de la paroi du corps gonflable qui est retourné par le corps gonflable. Le récepteur est configuré pour compter le temps écoulé entre l'émission du signal ultrason et sa réception pour en déduire une distance.

Selon encore un autre exemple, le capteur de distance 6' est un capteur laser. Le capteur laser est un capteur de « temps de vol » comprenant un émetteur et un récepteur.

L'émetteur est configuré pour émettre un rayonnement laser vers la paroi du corps gonflable qui est retourné par le corps gonflable. Le récepteur est configuré pour compter le temps écoulé entre l'émission du rayonnement laser et sa réception pour en déduire une distance.

Dans cet exemple le dispositif de mesure peut comprendre une diode électroluminescente (LED) destinée à fournir un éclairage dans l'intérieur du corps gonflable.

Dans l'exemple illustré à la figure 4 ledit au moins un capteur de distance est un appareil de prise de vue tel qu'une caméra ou un appareil photo digital 6" et le corps gonflable 2 comprend au moins un motif (non représenté) ou une nervure 18 agencé sur une face interne de la paroi du corps gonflable.

Le motif peut être par exemple une couleur ou une texture particulière aléatoire ou prédéterminée.

Le motif et la nervure sont destinés à permettre à l'appareil de prise de vue de détecter une distorsion du corps gonflable.

Dans l'exemple représenté à la figure 4, le corps gonflable 2 comprend une pluralité de nervures 18 agencées sur la face interne de la paroi du corps gonflable2.

L'appareil de prise de vue permet de connaitre, en temps réel, un état de la face interne de la paroi du corps gonflable. En comparant une image prise à un instant T1, par rapport à une image initiale prise à un temps T0, il est possible de déduire à l'aide d'une analyse d'image, l'augmentation de la dimension du corps gonflable. Par exemple le dispositif de mesure 6 peut comprendre une diode électroluminescente (LED) 30 destinée à fournir un éclairage dans l'intérieur du corps gonflable.

Dans les différents exemples décrits ci-dessus, les capteurs de distances sont reliés au microcontrôleur 8 du dispositif de contrôle au moyen de fils conducteurs 32.

Dans les exemples représentés aux figures 3 et 4, les fils conducteurs passent par la poignées 10.

Les capteurs de distances permettent de calculer un rayon du corps gonflable.

Le diamètre est déduit à partir du rayon calculé.

Il est à noter que le dispositif de mesure peut comprendre à cet effet une unité de traitement qui peut être distincte ou partagée avec le dispositif de contrôle

Selon un deuxième mode de réalisation illustré à la figure 5, le dispositif de mesure 6 est agencé en dehors du corps gonflable.

Dans ce mode de réalisation, le dispositif de gonflage 4 du corps gonflable doit comprendre le moteur 4" et l'électrovanne 14.

Dans ce mode de réalisation, le système comprend une première partie destinée à être placée dans les voies génitales et une deuxième partie destinée à rester en extérieur des voies génitales. La première partie comprend le corps gonflable 2 et la deuxième partie comprend le dispositif de gonflage 4, le dispositif de contrôle 8 et le dispositif de mesure 6.

Dans ce mode de réalisation le dispositif de mesure 6 comprend au moins un capteur de pression 34 relié au conduit 12 du système et au moins un calculateur 40 configuré pour calculer une quantité d'air entrant dans le corps gonflable et/ou sortant du corps gonflable. Le capteur de pression 34 est relié au conduit 12 du système 1.

Par exemple le calculateur 40 est configuré pour calculer une quantité d'air entrant dans le corps gonflable en utilisant des informations communiquées par le moteur 4".

Par exemple le calculateur 40 est configuré pour calculer une quantité d'air sortant du corps gonflable en utilisant des informations communiquées par l'électrovanne 14.

Le moteur 4", l'électrovanne 14 et le capteur de pression 34 sont reliés au dispositif de contrôle au moyen de fils conducteurs 35, 36, 37. Le calculateur 40 est relié au dispositif de contrôle 8, par exemple le dispositif de contrôle peut comprendre le calculateur 40.

Le dispositif de mesure peut comprendre un capteur de température (non représenté), relié au conduit 12 du système 1.

Le dispositif de mesure 6 est agencé pour déterminer la dimension du corps gonflable en fonction d'une valeur de pression, d'une valeur de température et d'une quantité d'air dans le corps gonflable.

Il est à noter que le dispositif de mesure peut comprendre à cet effet une unité de traitement qui peut être distincte ou partagée avec le dispositif de contrôle

Dans ce mode de réalisation, la dimension du corps gonflable est déterminée en s'appuyant sur la loi des gaz parfait et la relation PV=nRT dans laquelle :
- P correspond à la pression qui est déterminée au moyen du capteur de pression 34 ;
- T correspond à la température qui est déterminée soit au moyen d'un capteur de température, dans ce cas le dispositif de mesure comprend au moins un capteur de température (non représenté), soit par une estimation de la température ambiante :
- R correspond à la constante universelle des gaz parfait égale à 8,314 J mol⁻¹ K⁻¹
- n correspond à la quantité d'air entrant ou sortant du corps gonflable qui est calculée par le calculateur et qui est déterminée via des informations communiquées par le moteur (la durée d'allumage du moteur et son débit en fonction de la pression permet de connaitre la quantité d'air insufflée par le ballon) et/ou via des informations communiquées par l'électrovanne (la durée d'ouverture de l'électrovanne et la pression permet de connaitre la quantité d'air sortant du ballon, à déduire de la quantité d'air insufflée par le moteur).

Ces paramètres permettent de déduire le volume d'air présent dans le corps gonflable. De ce volume peut être déduit le diamètre du corps gonflable soit en connaissant la forme du corps gonflable soit empiriquement par une série de tests permettant de connaitre le comportement du corps gonflable.

Dans tous les modes de réalisation qui viennent d'être décrits, le système peut comprendre un moyen de préhension 40 (figure 1) destiné à permettre une optimisation du positionnement du corps gonflable dans les voies génitales de la femme et du retrait du corps gonflable. Par exemple le moyen de préhension étant un manche semi-rigide.

Selon un exemple, le manche semi-rigide est télescopique. Par exemple le manche peut présenter une longueur maximale de 40 cm en position déployée.

Bien évidemment, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Système (1) pour la préparation des voies génitales d'une femme avant un accouchement, le système (1) comprenant :
- un corps gonflable (2) destiné à être placé dans les voies génitales de la femme, le corps gonflable (2) comprenant une paroi (20) comportant une première extrémité(22) ouverte destinée à permettre une entrée d'air dans le corps gonflable ou une sortie d'air du corps gonflable et une deuxième extrémité (24) fermée opposée à la première extrémité (22),
- un dispositif de gonflage (4) du corps gonflable (2) relié fluidiquement à la première extrémité (22) ouverte du corps gonflable (2) par un conduit (12).
- un dispositif de mesure (6) configuré pour mesurer une dimension du corps gonflable,
- un dispositif de contrôle (8) configuré pour collecter les données de mesure de dimension du dispositif de mesure (6),
lorsque le corps est gonflé, le corps gonflable (2) présente une section transversale (T) sensiblement circulaire et permet de simuler un passage d'une tête d'un bébé.

2. Système selon la revendication 1, dans lequel le dispositif de contrôle (8) est agencé pour transmette les données de mesure de dimension du dispositif de mesure (6) à un dispositif d'interface configuré pour recevoir et afficher des données de mesure de dimension du dispositif de mesure, notamment par une liaison de communication sans fil.

3. Système selon l'une quelconque des revendications 1 ou 2 dans lequel le dispositif de mesure (6) est configuré pour mesurer une dimension du corps gonflable (2) correspondant à une section transversale du corps gonflable (2).

4. Système selon la revendication précédente dans lequel le système (1) étant agencé pour déterminer un diamètre de ladite section transversale du corps gonflable.

5. Système (1) selon l'une quelconques des revendications 1 à 4 dans lequel le dispositif de mesure (6) est intégré dans le corps gonflable et comprend au moins un capteur de distance (6', 6"), le corps gonflable (2) comprenant un support central (200) s'étendant dans le corps gonflable (2) et ledit au moins un capteur de distance (6', 6")étant agencé sur le support central du corps gonflable (2), le dispositif de mesure (6) étant configuré pour mesurer une distance entre le support central (200) et un point localisé sur une face interne de la paroi (20) du corps gonflable (2).

6. Système (1) selon la revendication précédente dans lequel le support central (200) est une tige semi-rigide et en ce que ledit au moins un capteur (6', 6") est agencé sur une extrémité libre (202) de la tige semi-rigide.

7. Système (1) selon l'une des revendications 5 ou 6 dans lequel ledit au moins un capteur de distance (6', 6") est un capteur choisi parmi le groupe constitué par un capteur infrarouge, un capteur ultrason et un capteur laser.

8. Système (1) selon l'une des revendications 5 ou 6 dans lequel ledit au moins un capteur de distance est un appareil de prise de vue (6") et en ce que le corps gonflable (2) comprend au moins un motif ou une nervure agencé sur une face interne de la paroi du corps gonflable (2).

9. Système (1) selon l'une quelconques des revendications 1 à 4 dans lequel le système (1) comprend un première partie destinée à être placée dans les voies génitales et une deuxième partie destinée à rester en extérieur des voies génitales, la première partie comprenant le corps gonflable (2) et la deuxième partie comprenant le dispositif de gonflage (4), le dispositif de contrôle (8) et le dispositif de mesure (6), le dispositif de mesure (6) comprenant au moins un capteur de pression (34), et au moins un calculateur (40) configuré pour calculer une quantité d'air entrant dans le corps gonflable et/ou sortant du corps gonflable.

10. Système selon la revendication précédente dans lequel le calculateur (40) est configuré pour calculer une quantité d'air entrant dans le corps gonflable utilisant des informations communiquées par un moteur (4").

11. Système (1) selon l'une des revendications 9 ou 10 dans lequel le calculateur (40) est configuré pour calculer une quantité d'air sortant du corps gonflable utilisant des informations communiquées par une électrovanne (12).

12. Système (1) selon l'une quelconques des revendications précédentes dans lequel la deuxième extrémité (24) du corps gonflable comprend un renflement (25).

13. Système (1) selon l'une quelconques des revendications précédentes dans lequel le système (1) comprend un moyen de préhension (40) destiné à permettre un positionnement du corps gonflable dans les voies génitales de la femme, le moyen de préhension étant un manche semi-rigide.

14. Système (1) selon la revendication précédente dans lequel le manche semi-rigide (40) est télescopique.

## Patentansprüche

1. System (1) zur Vorbereitung des Genitaltrakts einer Frau vor der Entbindung, wobei das System (1) umfasst:
- einen aufblasbaren Körper (2), der dazu bestimmt ist, im Genitaltrakt der Frau platziert zu werden, wobei der aufblasbare Körper (2) eine Wand (20) umfasst, die ein erstes offenes Ende (22), das dazu bestimmt ist, einen Lufteintritt in den aufblasbaren Körper oder einen Luftaustritt aus dem aufblasbaren Körper zu ermöglichen, und ein zweites geschlossenes Ende (24) gegenüber dem ersten Ende (22) einschließt,
- eine Aufblasvorrichtung (4) des aufblasbaren Körpers (2), die durch einen Kanal (12) fluidtechnisch mit dem ersten offenen Ende (22) des aufblasbaren Körpers (2) verbunden ist.
- eine Messvorrichtung (6), die ausgebildet ist, um eine Abmessung des aufblasbaren Körpers zu messen,
- eine Steuervorrichtung (8), die ausgebildet ist, um die Abmessungsmessdaten der Messvorrichtung (6) zu erheben,
wenn der Körper aufgeblasen ist, weist der aufblasbare Körper (2) einen im Wesentlichen kreisförmigen Querschnitt (T) auf und ermöglicht es, einen Durchgang eines Kopfes eines Babys zu simulieren.

2. System nach Anspruch 1, wobei die Steuervorrichtung (8) angeordnet ist, um die Abmessungsmessdaten der Messvorrichtung (6) an eine Schnittstellenvorrichtung zu übertragen, die ausgebildet ist, um Abmessungsmessdaten der Messvorrichtung, insbesondere über eine drahtlose Kommunikationsverbindung zu empfangen und anzuzeigen.

3. System nach einem der Ansprüche 1 oder 2, wobei die Messvorrichtung (6) ausgebildet ist, um eine Abmessung des aufblasbaren Körpers (2) zu messen, die einem Querschnitt des aufblasbaren Körpers (2) entspricht.

4. System nach dem vorstehenden Anspruch, wobei das System (1) angeordnet ist, um einen Durchmesser des Querschnitts des aufblasbaren Körpers zu bestimmen.

5. System (1) nach einem der Ansprüche 1 bis 4, wobei die Messvorrichtung (6) in den aufblasbaren Körper integriert ist und mindestens einen Abstandssensor (6', 6") umfasst, wobei der aufblasbare Körper (2) eine zentrale Stütze (200) umfasst, die sich in dem aufblasbaren Körper (2) erstreckt, und der mindestens eine Abstandssensor (6', 6") auf der zentralen Stütze des aufblasbaren Körpers (2) angeordnet ist, wobei die Messvorrichtung (6) ausgebildet ist, um einen Abstand zwischen der zentralen Stütze (200) und einem Punkt zu messen, der sich an einer Innenfläche der Wand (20) des aufblasbaren Körpers (2) befindet.

6. System (1) nach dem vorstehenden Anspruch, wobei die zentrale Stütze (200) ein halbstarrer Stab ist und dadurch, dass der mindestens eine Sensor (6', 6") an einem freien Ende (202) des halbstarren Stabs angeordnet ist.

7. System (1) nach einem der Ansprüche 5 oder 6, wobei der mindestens eine Abstandssensor (6', 6") ein Sensor ist, der aus der Gruppe ausgewählt ist, die aus einem Infrarotsensor, einem Ultraschallsensor und einem Lasersensor besteht.

8. System (1) nach einem der Ansprüche 5 oder 6, wobei der wenigstens eine Abstandssensor ein Bildaufnahmegerät (6") ist und dadurch, dass der aufblasbare Körper (2) mindestens ein Motiv oder eine Rippe umfasst, die auf einer Innenfläche der Wand des aufblasbaren Körpers (2) angeordnet ist.

9. System (1) nach einem der Ansprüche 1 bis 4, wobei das System (1) einen ersten Teil umfasst, der dazu bestimmt ist, im Genitaltrakt platziert zu werden, und einen zweiten Teil, der dazu bestimmt ist, außerhalb des Genitaltrakts zu bleiben, wobei der erste Teil den aufblasbaren Körper (2) umfasst und der zweite Teil die Aufblasvorrichtung (4), die Steuervorrichtung (8) und die Messvorrichtung (6) umfasst, wobei die Messvorrichtung (6) mindestens einen Drucksensor (34) umfasst, und mindestens einen Rechner (40) umfasst, der ausgebildet ist, um eine Luftmenge zu berechnen, die in den aufblasbaren Körper eintritt und/oder aus dem aufblasbaren Körper austritt.

10. System nach dem vorstehenden Anspruch, wobei der Rechner (40) ausgebildet ist, um eine Luftmenge, die in den aufblasbaren Körper eintritt, unter Verwendung von Informationen, die von einem Motor (4") übermittelt werden, zu berechnen.

11. System (1) nach einem der Ansprüche 9 oder 10, wobei der Rechner (40) ausgebildet ist, um eine Luftmenge, die aus dem aufblasbaren Körper austritt, unter Verwendung von Informationen zu berechnen, die von einem Magnetventil (12) übermittelt werden.

12. System (1) nach einem der vorstehenden Ansprüche, wobei das zweite Ende (24) des aufblasbaren Körpers einen Wulst (25) umfasst.

13. System (1) nach einem der vorstehenden Ansprüche, wobei das System (1) ein Greifmittel (40) umfasst, das dazu bestimmt ist, eine Positionierung des aufblasbaren Körpers im Genitaltrakt der Frau zu ermöglichen, wobei das Greifmittel ein halbstarrer Griff ist.

14. System (1) nach dem vorstehenden Anspruch, wobei der halbstarre Griff (40) teleskopisch ist.

## Claims

1. A system (1) for preparing the genital tract of a woman before a childbirth, the system (1) comprising:
- an inflatable body (2) intended to be placed in the genital tract of the woman, the inflatable body (2) comprising a wall (20) including a first open end (22) intended to allow air to enter the body inflatable or an air to leave the inflatable body and a second closed end (24) opposite the first end (22),
- an inflation device (4) of the inflatable body (2) fluidly connected to the first open end (22) of the inflatable body (2) by a conduit (12).
- a measuring device (6) configured to measure a dimension of the inflatable body,
- a control device (8) configured to collect the dimension measurement data of the measuring device (6),
when the body is inflated, the inflatable body (2) has a substantially circular cross section (T) and makes it possible to simulate a passage of a baby's head.

2. The system according to claim 1, wherein the control device (8) is arranged to transmit dimension measurement data from the measuring device (6) to an interface device configured to receive and display dimension measurement data of the measuring device, in particular by a wireless communication link.

3. The system according to any of claims 1 or 2 wherein the measuring device (6) is configured to measure a dimension of the inflatable body (2) corresponding to a cross section of the inflatable body (2).

4. The system according to the preceding claim wherein the system (1) being arranged to determine a diameter of said cross section of the inflatable body.

5. The system (1) according to any one of claims 1 to 4 wherein the measuring device (6) is integrated into the inflatable body and comprises at least one distance sensor (6', 6"), the inflatable body (2) comprising a central support (200) extending into the inflatable body (2) and said at least one distance sensor (6', 6"') being arranged on the central support of the inflatable body (2), the measuring device (6) being configured to measure a distance between the central support (200) and a point located on an inner face of the wall (20) of the inflatable body (2).

6. The system (1) according to the preceding claim wherein the central support (200) is a semi-rigid rod and in that said at least one sensor (6', 6"') is arranged on a free end (202) of the semi-rigid rod.

7. The system (1) according to any of claims 5 or 6 wherein said at least one distance sensor (6', 6"") is a sensor selected from the group consisting of an infrared sensor, an ultrasonic sensor and a laser sensor.

8. The system (1) according to any of claims 5 or 6 wherein said at least one distance sensor is a shooting device (6"') and in that the inflatable body (2) comprises at least one pattern or a rib arranged on an inner face of the wall of the inflatable body (2).

9. The system (1) according to any one of claims 1 to 4 wherein the system (1) comprises a first part intended to be placed in the genital tract and a second part intended to remain outside the genital tract, the first part comprising the inflatable body (2) and the second part comprising the inflation device (4), the control device (8) and the measuring device (6), the measuring device (6) comprising at least one pressure sensor (34), and at least one computer (40) configured to calculate a quantity of air entering the inflatable body and/or leaving the inflatable body.

10. The system according to the preceding claim wherein the computer (40) is configured to calculate a quantity of air entering the inflatable body using information communicated by a motor (4").

11. The system (1) according to any of claims 9 or 10 wherein the computer (40) is configured to calculate a quantity of air leaving the inflatable body using information communicated by a solenoid valve (12).

12. The system (1) according to any one of the preceding claims wherein the second end (24) of the inflatable body comprises a bulge (25).

13. The system (1) according to any one of the preceding claims wherein the system (1) comprises a gripping means (40) intended to allow a positioning of the inflatable body in the genital tract of the woman, the gripping means being a semi-rigid handle.

14. The system (1) according to the preceding claim wherein the semi-rigid handle (40) is telescopic.
